# EUROPEAN PATENT APPLICATION

(11) **EP 0 879 884 A2**
(43) Date of publication of application: **25.11.1998**
(21) Application number: 98109208.3
(22) Date of filing: 20.05.1998
(51) Int. Cl.: C12N 15/12, C12N 5/10, C07K 14/73, A61K 38/17

(54) **Novel CD4 derivatives**

(30) Priority: 20.05.1997 EP 97108162
(71) Applicant: Kufer, Peter, Dr., 85368 Moosburg (DE)
(72) Inventor: Kufer, Peter, D-85368 Moosburg (DE); Bastani, Kaveh, D-80796 München (DE)
(74) Representative: VOSSIUS & PARTNER

(57) **Abstract**

The present invention relates to nucleic acid molecules encoding polypeptides that are derived from the human CD4. These polypeptides display an increased affinity to glycoprotein 120 of HIV. Additionally, and preferably, said polypeptides also decrease or avoid the induction of binding sites for HIV-co-receptors on gp120 and/or on the protein complex consisting of gp120 and said CD4-derived polypeptide. The present invention also relates to pharmaceutical and diagnostic compositions comprising said nucleic acids, vectors comprising said nucleic acids and/or said polypeptides. Finally, the present invention relates to methods for selecting polypeptides having the above-recited advantageous properties.

## Description

The present invention relates to nucleic acid molecules encoding polypeptides that are derived from the human CD4 molecule. These polypeptides display an increased affinity to glycoprotein 120 of HIV. Additionally, and preferably, said polypeptides also decrease or avoid the induction of binding sites for HIV-co-receptors on gp120 and/or on the protein complex consisting of gp120 and said CD4-derived polypeptides. The present invention also relates to pharmaceutical and diagnostic compositions comprising said nucleic acids, vectors comprising said nucleic acids and/or said polypeptides. Finally, the present invention relates to methods for selecting polypeptides having the above-recited advantageous properties.

A common feature of different HIV-types and HIV-strains is an infection of CD4-positive cells. The CD4 molecule on the surface of human cells is the primary receptor for HIV (Klatzmann et al., Nature 312 (1984), 767-768). This CD4 molecule is only expressed by cells of hematological origin and by cells of the immune system. Furthermore, only this type of cell can be infected by HIV. This tropism of HIV is most likely the main cause for the pathogenicity in the usually deadly cause of the disease (Capon & Ward, Ann. Rev. Immunol. 9(1991), 649-678).

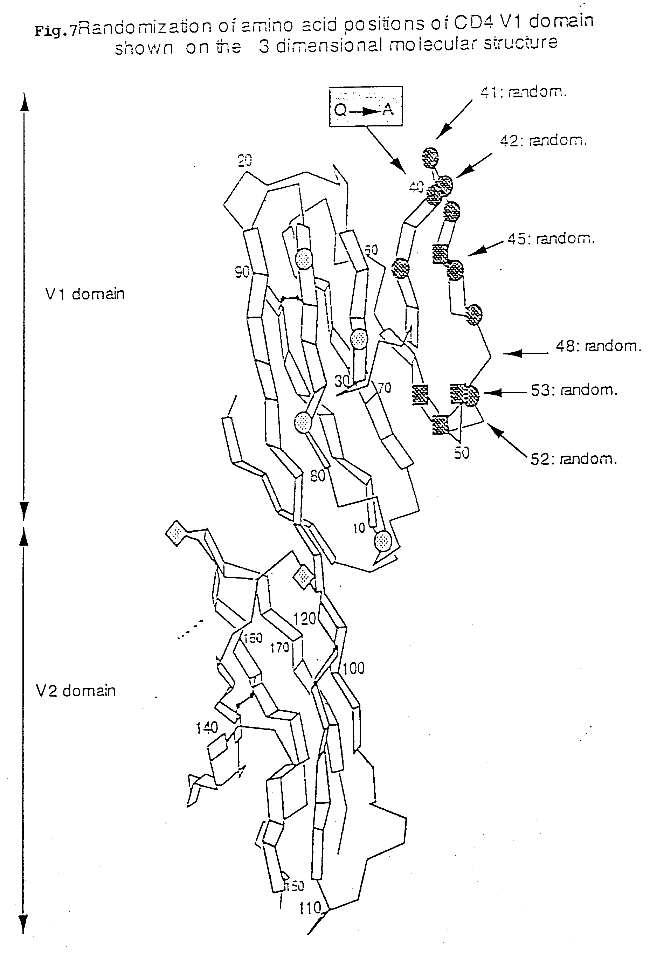

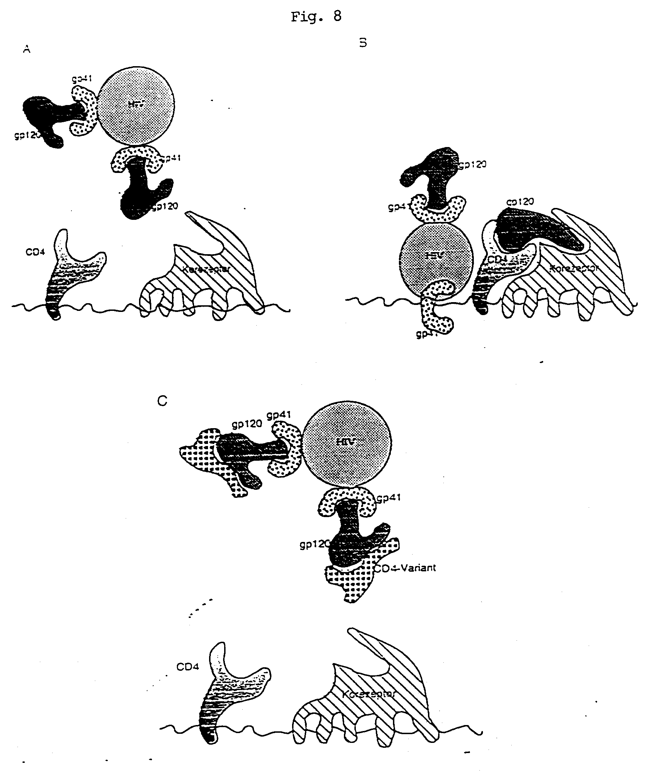

On the basis of these results, it cannot be excluded that soluble CD4 molecules can prevent or at least reduce the risk of an HIV infection. Under in vitro conditions, soluble CD4 could efficiently block the infection by HIV (Deen et al., Nature 331 (1988), 82-84; Smith et al., Science 238 (1987), 1704-1704). However, additional experiments demonstrated that the primary HIV isolates which were not adapted to in vitro conditions could not be efficiently inhibited by soluble CD4 (Ashkenazi et al., Proc. Natl. Acad. Sci. USA 88 (1991), 7056-7060). Today it is known that binding of CD4 to the HIV envelope protein gp120 induces the binding of secondary receptors (co-receptors) to gp120 (Fig. 7) (Berson et al., J. Biol. Sep. 70 (1996), 6288-6295; Deng et al., Nature 381 (1996), 661-666), Lapham et al., Science 274 (1996), 602-605, Wu et al., Nature 384 (1996), 179-183, Trkola et al., Nature 384 (1996), 184-187. This data may explain why soluble CD4 did not inhibit primary HIV isolates in vitro. Binding of gp120 to different co-receptors which, as is known so far, all belong to the group of chemokine receptors results in entry of the virus into the cell (Alkhatib et al., Science 272 (1996), 1955-1958). Soluble CD4 molecules could therefore induce binding of gp120 to said co-receptors and thus lead to the infection of co-receptor bearing cells.

From the prior art, it is known that the first extracellular domain of CD4 (V1-domain) binds with a high affinity to gp120 (Arthos et al., Cell 57 (1989), 469-481; Chao et al., J. Biol. Chem. 264 (1989), 5812-5817). In particular, amino acid positions between amino acids numbers 40 and 55 of the CD4-V1-domain appear to be relevant for binding to gp120 (Clayton et al., Nature 335 (1988), 363-366; Landau et al., Nature 334 (1988), 159-162; Mizukami et al., Proc. Natl. Acad. Sci. USA 85 (1988), 9273-9277). Rat CD4 mutants comprising the human CD4 sequence in the region of 33 to 62 can inhibit HIV infection of HeLa cells (Simon et al., J. Exp. Med. 177 (1993), 949-954). There are detailed experimental data to date, as to whether the exchange of single amino acids in this region leads to a destruction or at least reduction or, alternatively, to an enhancement of CD4 binding to gp120 (Arthos et al., 757 (1989), 469-481; Ashkenazi et al., Proc. Natl. Acad. Sci. USA 87 (1990), 7150-7154). One of the known data is that the exchange of glutamine in position 40 by alanine enhances binding of CD4 to gp120 by a factor of 2 (Ashkenazi et al., loc. cit.).

The above prior art data have, so far, not resulted in the development of a CD4-based medicament that leads to an enhanced binding of CD4 to gp120 which in turn would result in a significant decrease of the virus load in infected humans. Additionally and preferably, it is desirable to select for CD4 derivatives that are capable of decreasing or avoiding the induction of binding sites for HIV co-receptors of gp120 and/or the protein complex consisting of gp120 and said CD4-derived polypeptide. Accordingly, the technical problem underlying the present invention was to provide molecules that fulfill these requirements and are therapeutically useful. The solution to said technical problem is achieved by providing the embodiments characterized in the claims.

Accordingly, the present invention relates to a nucleic acid molecule encoding a polypeptide being derived from the human CD4 molecule but differing therefrom by (a) a tryptophane or tyrosine residue or another aromatic amino acid instead of a threonine residue in position 45.
In accordance with the present invention, the term "a polypeptide being derived from the human CD4 molecule" is intended to mean any polypeptide that has the function of binding to gp120 of HIV such as HIV-1 or HIV-2 and comprises preferably at least the V1-domain of CD4. In further embodiments, this term relates to parts of said V1 domain necessary and sufficient to bind to gp120. Further in accordance with the present invention, said V1 domain may be altered insofar that binding to gp120 is improved by replacing one or more amino acids which preferably contribute to binding of CD4 to gp120. The overall three-dimensional structure of at least the V1 domain of CD4 should, however, be retained.It is further envisaged that other fragments of CD4 are covered by the above-recited term such as fragments comprising the V1 and V2 domains of CD4. The above-recited term further comprises fusion proteins of CD4 or fragments thereof with different (poly)peptides. These different (poly)peptides may be advantageously immunoglobulin domains or parts thereof. Thus, fusion of V1 domains with constant regions of IgG-heavy chain gives rise to a molecule that has two binding sites for gp120. In other words, this and similar constructs give rise to multimerization devices that have particular advantages when used as therapeutic agents. Additionally, the above recited term comprises said altered CD4 molecules or fragments thereof that are chemically covalently or non-covalently bound to unrelated molecules having e.g. carrier or therapeutic functions such as immunoglobulin molecules or domains thereof.

The exchange of amino acids that are replaced in the CD4 derivatives may be effected according to conventional protocols. Changes on the level of the nucleic acids may be effected using PCR-based randomization of selected amino acid positions (Barbas, Proc. Natl. Acad. Sci. USA 89 (1992), 4457-4461).

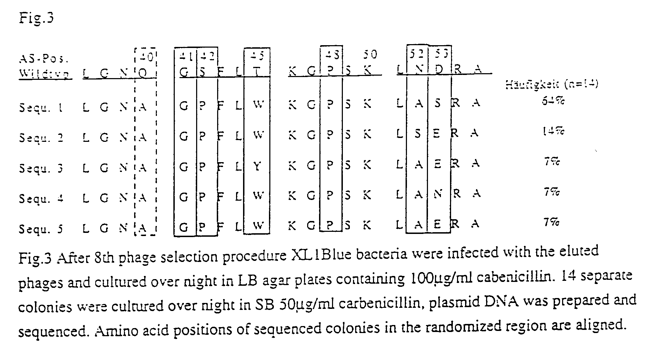

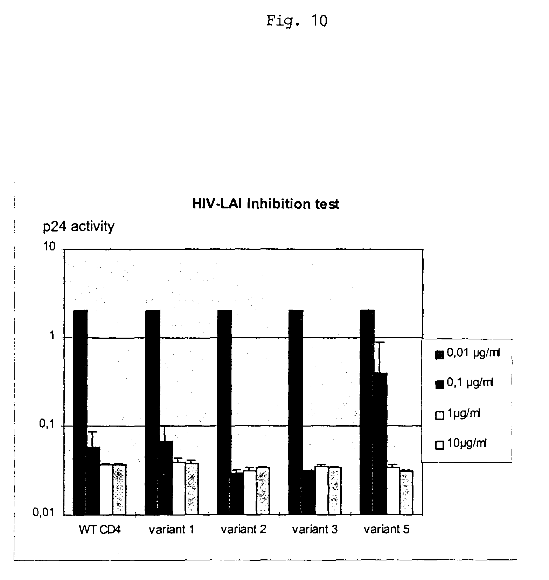

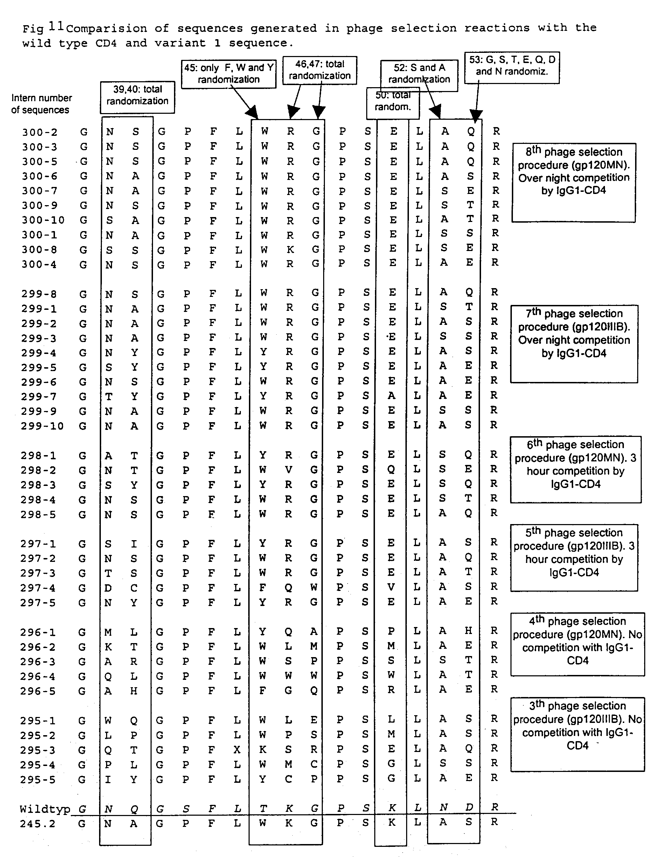

In accordance with the present invention, it was reasoned that in particular amino acid positions 39, 40, 41, 42, 45, 46, 47, 48, 50, 52 and 53, (Fig. 3 and Fig. 11) which are non-conserved amino acids in CD4-molecules of different mammalian species, may be useful for testing the effect of an exchange of said amino acids with regard to the binding properties of CD4 to gp120. It was furthermore expected that an exchange of these amino acids by different ones would not result in an overall destruction of the three-dimensional structure of the CD4 derivatives. Using degenerate PCR primers, the above-recited positions were randomized starting from molecules in which wild-type glutamine 40 was replaced by alanine. Applying this strategy, a DNA diversity of about 10⁹ and an amino acid diversity of about 10⁸ was obtained. Using special and, for the purposes of the present invention, advantageous vectors (pComb3H) and helper phages (VCSM13), CD4 derivatives which comprise the V1 domain were expressed as a part of the coat protein III of bacteriophage M13 (Gram et al., Proc. Natl. Acad. Sci. USA 89 (1992), 3576-3580). The genetic code of each special CD4 variant is in this method contained in the respective bacteriophage. The resulting bacteriophage can be concentrated to a density of 10¹² per ml. gp120 may be bound to a solid phase and subsequently incubated with the concentrated bacteriophage library. The bound bacteriophage are subsequently specifically eluted and after infection of E. coli bacteria reamplified. In particular, the two or more fold repetition of this procedure results in the isolation of CD4 derivatives comprising the V1 domain with a high affinity to gp120.

Applying this strategy, it was surprisingly found that the replacement of the amino acid threonine in position 45 by an aromatic amino acid and preferably by tryptophane or tyrosine results in a CD4 derivative displaying a high affinity to gp120. This result is in particular unexpected since a large number of mutational experiments using the region between amino acids 36 and 57 of CD4 have already been carried out without, however, providing a result that was obtained in accordance with the present invention; see, for example, Roberts et al., Meth. Enzymol. 267 (1996), 68-82. In accordance with the present invention, it is also envisaged that the polypeptide encoded by the nucleic acid molecule of the invention results in the decrease or preferably avoidance of the induction of binding sites for HIV co-receptors on gp120 and/or on the protein complex consisting of gp120 and said CD4 derivatives. Alternatively, said CD4 derivatives provide a basis for the selection of such molecules by, for example, further rounds of mutagenesis.

Preferably, said nucleic acid molecules also differ from the human CD4 molecule by (b) a proline residue instead of a serine residue in position 42.

In an additional preferred embodiment of the invention, said nucleic acid molecule encodes a polypeptide that further differs from said human CD4 molecule by (c) a replacement of the amino acid asparagine in position 52 by a different amino acid. Said different amino acid is advantageously a small amino acid.

Most preferably said amino acid in position 52 is alanine or serine.

In an additional preferred embodiment of the invention, said polypeptide further differs from said human CD4 molecule by (d) a replacement of the amino acid aspartic acid in position 53 by a different amino acid.
Advantageously, said different amino acid is glycine, serine, threonine, asparagine, glutamic acid or glutamine

An additional preferred embodiment of the present invention relates to a nucleic acid molecule wherein said polypeptide is further characterized by asparagine, serine or threonine in position 39, an alanine, serine or tyrosine residue in position 40, a glycine residue in position 41, arginine in position 46, a glycine residue in position 47, a proline residue in position 48, and/or glutamic acid or alanine in position 50 including any combinations thereof.

Preferably, the nucleic acid molecule according to the invention encodes a polypeptide that further differs from human CD4 in that a sequence comparable to an immunoglobulin domain hypervariable loop is inserted into at least one V-κ-CDR homologous region of the CD4 molecule.
The V-κ-CDR homologous region of CD4 is described in Wang et aL., Nature 348 (1990), 411-418 and is advantageously homologous to the CDR-1 or the CDR-3 of the kappa light chain variable domain.
This embodiment of the invention is particularly advantageous since it can be used to dramatically increase the binding capacity of soluble CD4 molecules to gp120. In addition, the inclusion of said loop, after an appropriate selection step, is expected to significantly contribute to the decrease and preferably the avoidance of the induction of binding sites for HIV co-receptors on gp120 and/or on the protein complex consisting of gp120 and the CD4 derivative of the invention. The immunoglobulin-like hypervariable loop, after insertion into said at least one V-κ-CDR homologous region may be mutagenized according to conventional protocols and selected for increased binding to gp120 and for the above-recited decrease or avoidance of said induction by methods that are known in the art. The hypervariable loop may vary in length and/or sequence from a naturally occurring loop both prior to and after the mutagenesis step. The phage display method that is advantageously used for selecting appropriate polypeptides has the particular advantage that extremely large numbers of clones can be tested for their properties.

Another preferred embodiment of the invention relates to nucleic acid molecules which are DNA or RNA.
In the case of DNA, the nucleic acid molecules are advantageously cDNA, synthetic DNA, semi-synthetic DNA or genomic DNA.

The invention further relates to a vector comprising the nucleic acid molecule of the invention. The vector of the invention may advantageously be an expression vector. Also preferred are vectors that are useful for gene therapy. For example, the genomic information of the above-recited CD4 derivatives may be used for the intracellular prevention of the production of new HIV in certain target cells of an organism. This can, for example, be effected by the intracellular production of CD4 derivatives under the control of an HIV-LTR in CD4 positive cells. Another possibility for the use of the vector of the invention in gene therapy is the in vivo stimulation of autologous cells for the synthesis and release of soluble CD4 derivatives. These derivatives would then prevent or at least significantly reduce the infection of further cells by HIV in the bloodstream and/or lymphoid tissue. A further option for the use of the vector of the invention in gene therapy is the generation of preferably autologous hematopoietic stem cells that will differentiate into CD4 positive cells, preferably T cells and mononuclear phagocytes that instead of wild-type CD4 express a CD4 dervative of the invention making them reistant or at least significantly less susceptible to HIV infection.

The present invention also relates to host cells transformed with the vector of the invention. Said host cells may be bacterial cells and preferably E. coli cells. Alternatively, said hosts may be eucaryotic cells and preferably yeast cells, insect cells that may be infected with baculoviruses or mammalian cells, for example, non-transformed primary human cells. The latter cells may be advantageously used for gene therapy. In a further embodiment, said hosts may be transgenic animals or plants which may be used for the production of the polypeptide of the invention.

Additionally, the present invention relates to a method of producing a polypeptide encoded by the nucleic acid molecule of the invention comprising culturing the host according to the invention and recovering the produced polypeptide. The details of the method of production, for example, the composition of the culturing medium will eventually depend on the host that is used for producing the polypeptide. If cells are used as host organisms and said host organisms are cultured in liquid medium, the polypeptide may, depending on the specific vector construction and the host cell used, either be exported into the medium or retained in the cell or both. The person skilled in the art will, of course, take these prerequisites into account when recovering the polypeptide of the invention.

Additionally, the present invention relates to a polypeptide encoded by the nucleic acid of the invention or produced by the method of the invention.

The present invention also relates to a pharmaceutical composition comprising the nucleic acid according to the invention, the vector of the invention and/or the polypeptide of the invention and optionally a pharmaceutically acceptable carrier and/or diluent. The pharmaceutical composition of the invention may be advantageously used for preventing or treating HIV infections. The dosage regimen will be determined by the attending physician considering the condition of the patient, the severity of the disease and other clinical factors. Generally, the regimen as a regular and administration of the pharmaceutical composition will depend on the actually pharmaceutically active ingredient that is contained in the pharmaceutical composition. In the case of the polypeptide of the invention, it would usually be in the range of 100mg or less per kg of body weight per day. Administration of the suitable compositions may be effected in different ways, e.g., by intravenous, intraperitoneal, subcuteneous, intramuscular or other routes.

Preferably, the pharmaceutical composition of the invention is a vaccine.

Again, the formulation of vaccines may be effected according to routine procedures.

The present invention also relates to diagnostic compositions comprising the nucleic acid of the invention, the vector of the invention and/or the polypeptide according to the invention.
The diagnostic composition of the invention can be advantageously employed for specifically capturing and thus enriching HIV particles from samples, for example, blood samples, of HIV patients. The enrichment may be followed by a highly sensitive determination of the viral load of HIV in the patient. Additionally, the diagnostic composition of the invention may be used for measuring soluble gp120 protein in samples from a patient. It may further be used for the ex vivo or in vivo diagnosis of organs and tissues with high gp120 expression. The result of such a diagnosis would indicate HIV replication by in-vivo targeting and subsequent imaging.

The invention further relates to a method of selecting a polypeptide being derived from human CD4 and having an enhanced affinity to gp120 comprising:
(a) mutating at least one amino acid not essential for the overall conformation of CD4 in the region of the polypeptide encoded by the nucleic acid molecule of the invention corresponding to amino acids 36 to 57 of the human CD4 molecule with the proviso that the amino acids in positions 41, 45 and 48, after the mutation step, are glycine, an aromatic amino acid and proline, respectively; and
(b) evaluating whether the affinity of the mutated polypeptide displays an increased affinity for gp120 of HIV as compared to a reference polypeptide which is preferably the polypeptide of the invention.
The method of the invention can be advantageously used for selecting CD4 molecules or further mutating CD4 derivatives and isolating novel molecules that have a high affinity to gp120 than previously known reference molecules. The high affinity binding of the CD4 derivatives of the invention has a significant therapeutic impact. In particular, it is envisaged that the presence of tightly binding soluble CD4 derivates in the bloodstream or lymphoid tissue will inhibit the binding of viral gp120 protein to CD4 molecules on immunocompetent or hematopoietic cells due to the lower affinity of said gp120 to said CD4 molecules on said cells.
Said mutation step (a) may be effected by using PCR-based randomization of selected amino acid positions and the subsequent in vitro selection and evaluation step may be done using an in vitro selection assay for binding to gp120 preferably by employing the phage display method.

Additionally, the present invention relates to a method of selecting a polypeptide being derived from human CD4 and having the capacity to significantly decrease and preferably avoid the induction of binding sites for HIV-co-receptors on gp120 and/or on the protein complex consisting of gp120 and said CD4-derived polypeptide comprising:
(a) mutating at least one amino acid not essential for the overall conformation of CD4 in the region of the polypeptide encoded by the nucleic acid molecule of the invention corresponding to amino acids 36 to 57 of the human CD4 molecule with the proviso that the amino acids in positions 41, 45 and 48, after the mutation step, are glycine, an aromatic amino acid and proline, respectively; and
(b) evaluating whether the mutated polypeptide displays the property of decreasing and preferably avoiding the induction of binding sites for HIV-co-receptors on gp120 and/or on the protein complex consisting of gp120 and said CD4-derived polypeptide as compared to a reference polypeptide which is preferably the polypeptide of the invention.
In the above recited methods, the reference polypeptide may , e.g., be a native CD4 molecule.
Again, this embodiment bears a significant therapeutic impact. This is because it is known to date that the binding of gp120 to CD4 is not sufficient to mediate HIV entry into the cells. Rather, binding of gp120 to CD4 induces binding sites for HIV co-receptors on gp120 and/or on the protein complex consisting of gp120 and the CD4 molecule. By altering the natural CD4 molecule in such a way that induction is decreased or inhibited, entry of the HIV into the target cell is avoided or at least significantly reduced.

Preferably, said method of the invention further comprises the steps of
(c) testing the effect of a sequence comparable to an immunoglobulin domain hypervariable loop which is inserted into said at least one V-kappa-CDR homologous region of the CD4 molecule of said polypeptide on the affinity and/or the decrease and preferably avoidance of the induction of binding sites for HIV-co-receptors on gp120 and/or on the protein complex consisting of gp120 and said CD4-derived polypeptide; and
(d) selecting for polypeptides having an increased affinity and/or displaying a decrease and preferably avoidance of the induction of binding sites for HIV-co-receptors on gp120 and/or on the protein complex consisting of gp120 and said CD4-derived polypeptide.
The term " a sequence comparable to an immunoglobulin domain hypervariable loop" as used in connection with the present invention is intended to mean an amino acid sequence fulfilling or mediating functions of immunoglobulin hypervariable loops preferably by being capable of getting into physical contact with an interaction site on another molecule, said amino acid sequence not being limited to naturally occuring immunoglobulin domain hypervariable loops.
This embodiment of the invention is expected to be particularly useful for generating CD4 derivatives that inhibit or at least significantly decrease the induction of binding sites for HIV co-receptors.

Further preferred is a method wherein said evaluation and/or selection steps are effected with at least two gp120 molecules obtained from different HIV isolates. It is expected that the alternating or simultaneous selection using at least two gp120 molecules from different HIV isolates make the generation of escape mutants in an in vivo application less likely.

The CD4 derivatives produced in accordance with the method of the invention are accordingly expected to bind to a large number and preferably all naturally occurring HIV strains. Naturally, more than two gp120 molecules obtained from different HIV isolates may be used in the method of the invention.

It is further preferred that at least one of said gp120 molecules is gp120IIIB (gp120LAI) or gp120MN. Advantageously, both of said molecules are used in the selection/evaluation steps. Both isolates are well known in the art.
In all the above methods, the selection/evaluation may be effected using conventional methods such as ELISA methods and/or panning methods such as described in the appended examples. Advantageously, in addition to the usual acid based elution of bacteriophage carrying the polypeptide of the invention on their surface, an additional elution step using potassium rhodanide is employed. This step secures the elution of phages binding with an ultrahigh affinity.

Finally, the present invention relates to polypeptides that are obtainable by the method of the invention.

The figures show:
Figure 1:
   DNA sequence of the CD4V1 domain. Means PCR endonuclease restriction sites SacI, AvrII, XhoI, HindIII, XbaI and SpeI are added to this sequence. The amino acid positions 41, 42, 45, 48, 52 and 53 are randomized and a Q40A mutation is introduced.
Figure 2:
   Map of the phagemid plasmid pComb3H. The CD4-V1 domain is fused to a fragment of the M13 genIII. By infection of pComb3H transfectants with the helper phage VCSM13 CD4V1 can be displayed on the coat proteinIII
Figure 3:
   After 8th phage selection procedure XL1Blue bacteria were infected with the eluted phages and cultured over night in LB agar plates containing 100µg/ml cabenicillin. 14 separate colonies were cultured over night in SB 50µg/ml carbenicillin, plasmid DNA was prepared and sequenced. Amino acid positions of sequenced colonies in the randomized region are aligned.
Figure 4:
   Construction of human CD4V1V2-IgG1 fusion proteins. In BsrgI/MfeI restriction sites sequences of selected V1 mutants can be inserted.
Figure 5:
   Schematic visualization of the established fusion assay. For details see text of Example 4.
Figure 6:
   By co-expression of a HIV co-receptor (CXCR4 or CCR5) together with wild type CD4 or CD4 variants in the cell membrane of CHO cells an infection model for HIV can be established.
Figure 7:
   Randomization of amino acid positions of the CD4 V1 domain shown on the three-dimensional molecular structure.
Figure 8:
   Schematic representation of cell infection by HIV and of the inhibition of infection by CD4-derivatives.
   The HIV envelope protein gp120 is bound by non-covalent bonds to gp41 and has a strong affinity to CD4 (A). By binding of gp120 to CD4, the binding of HIV co-receptor to gp120 is induced. This results in the dissolution of gp120 from the viral surface. Subsequently, the interaction of gp41 with the cell membrane leads to virus fusion (B). The CD4-derivatives prepared in accordance with the present invention display, in contrast to wildtype CD4 the feature of not inducing, after binding to gp120, a binding of co-receptors on this molecule (C).
Figure 9:
   Fusion inducing capacity of CD4 and CD4 variant in the virus free system. CHO cells expressing CD4 or CD4 variants were cultured in 24 well plates. 2 days after cell confluence 160.000 P815 control cells or cells expressing envLAI or envMN were incubated together with CHO cells. The nutriant medium contained all selection supplements (dialysed FCS, dCF 0,1 µM, AAU (?), G418 1 mg/ml and hygromycine 300 µg/ml). After 10 days of co-culture cells were lysed and the luciferase activity was measured using a commercial kit supplied by Berthold company.
Figure 10:
   Neutralization of HIV-LAI by IgG1-CD4 and its derivatives. For the description of the experimental procedure see above. The p24 activity was measured by a commercial kit purchased from Boehringer Mannheim. The maximum of the p24 activity is 2 which is shown in a logarithmic scala.
Figure 11:
   Comparison of sequences generated in phage selection reactions with the wild type CD4 and variant 1 sequence.
Figure 12:
   PAGE-Analysis of purified IgG1-CD4 fusion protein and its derivatives. Lanes 1 and 8: Rainbow marker. Lane 7: human IgG as a contol. Lane 2 wild type IgG1-CD4. Lane 3-6 CD4 derivatives Variant 1, variant 2, variant 3 and variant 5.
Figure 13:
   Construction of MRG5 promoter dependent luciferase expression vector in pHygω. The MRG5 sequence consists of 5 GAL4 binding sites and a HIV1 TATA box.Abbreviations: TK= HSV1 thymidine kinase promoter. TK-Poly(A)= TK polyanenylation site. hyg= Hygromycine B resistance gene (Gene 25, Gritz-L et al.,179-88, 1983).

The examples illustrate the invention.

### Example 1: Construction of a phage library displaying CD4-V1 mutants

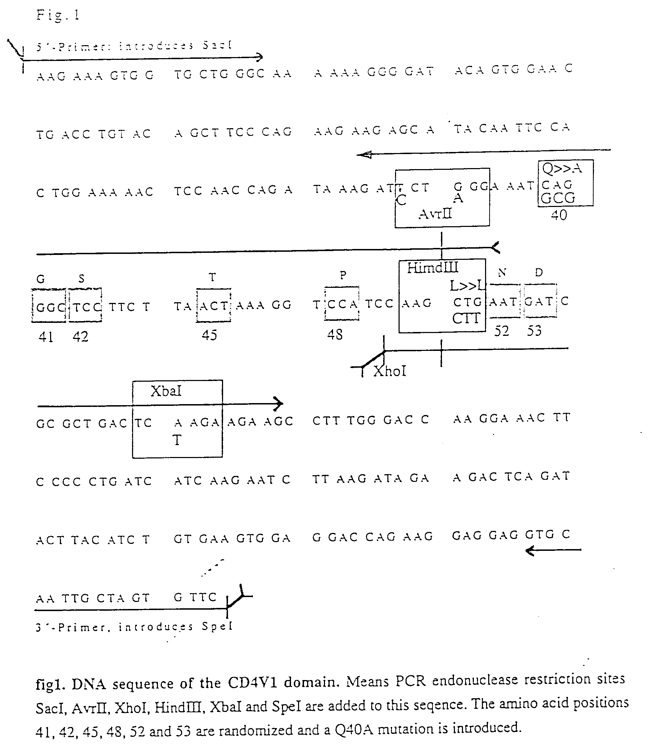

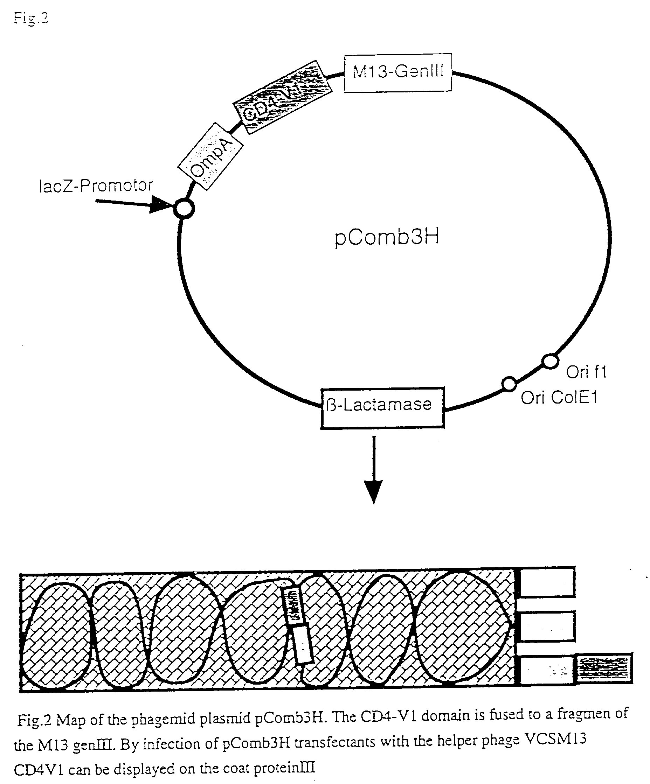

A phage library of human CD4-V1 mutants was constructed using degenerated primers in amino acid positions: 41, 42, 45, 48, 52, and 53 (Fig. 1). Amino acid positions are used according to Wang et al., loc. cit. The sequences of the used primers are shown in the Table 1. The randomization was performed in two steps. In the first step the positions 52 and 53 were randomized using a 3'-V1 primer that introduces a SpeI restriction site and the 5'-CDR2 primer that introduces a XhoI, a HindIII and a XbaI restriction site. Using a plasmid containing the full length cDNA of human CD4, a 176 bp DNA fragment was amplified by PCR. The used PCR protocol was: denaturation at 94°C, 5 minutes followed by 30 cycles of denaturation (94°C, 1 minute), primer annealing 52°C, 1 minute, and Taq polymerization (72°C, 30 seconds), followed by 5 minutes final polymerization at 72°C. The 176bp PCR products were purified by agarose gel electrophoresis and cut with restriction enzymes SpeI and XhoI. The digested DNA was purified again by agarose gel electrophoresis and ligated to the SpeI and XhoI restriction sites of the phagemid vector pComb3H (derived from pComb3, Barbas, Proc. Natl. Acad. Sci. U.S.A. 88, 1991, 7978-7982, Fig. 2). The resulting CD4-3'V1 library with 1024 different DNA species was transformed into electrocompetent E. coli XL1 blue by electroporation. More than 1x10⁸ bacterial transformants expressing the pComb3H amp gene and harbouring the inserted 3'V1 region were immediately generated after the transformation. Following over night growth of these bacteria, the plasmid DNA was purified and used for the generation of a V1 library in the second randomization step.

In order to randomize the positions 41, 42, 45 and 48, the 5'V1 primer introducing a Sac I restriction site and the 3'CDR2 primer introducing AvrII and HindIII restriction sites were used (Fig. 1, Table 1). Following PCR amplification (same conditions as in the step 1) the amplified 169 bp DNA was purified and digested with the restriction enzymes SacI and HindIII. This DNA was ligated with the 3'V1 plasmid library generated in the step 1 (also digested with the restriction enzymes SacI and HindIII) and introduced into the E. coli cells by electroporation. Multiple electroporations were performed and bacterial transformants were pooled. The number of transformants with the randomized CD4V1 region exceeded 2x10⁹ (the NNS-based randomization of 6 amino acid positions gives rise to 1x10⁹ possible DNA species).

The phagemid pComb3H contains the CD4-V1 region fused with a geneIII fragment of the single stranded bacteriophage M13. The complete product of geneIII is necessary for phage infectivity and is termed coat proteinIII (cpIII). The infection of the phagemid pcomb3H transformants with the helper bacteriophage VCSM13 (1x10¹² pfu per 100 ml bacterial culture) gives rise to infective M13 bacteriophages that express the CD4V1-cpIII fusion protein (Fig. 2). After overnight incubation at 37°C, phage particles were harvested from the bacterial supernatant by polyethylene glycol/NaCl precipitation. The phage library was redissolved in Tris buffered solution/1% bovine serum albumin (TBS/BSA) and incubated alternately with plastic bound gp120LAI or gp120MN at 37°C. Up to 10 washing steps were performed with a TBS-Tween® solution. Binding phages were eluted first with HCl/glycine, pH 2,2, and afterwards with 2,5M KSCN solution (=potassium rhodanid also named potassium thiocyanate). The additional elution with KSCN may be critical for the outcome of phage selection since some phages with high affinity to gp120 may not be eluted by acid treatment. These steps of phage propagation and selection were repeated 8 times (for input and output of phages see Table 2).
Unless otherwise mentioned, in vitro selection was carried out according to Burton, Proc. Natl. Acad. Sci. U.S.A. 88 (1991) 10134-10137. After the procedure of phage selection plasmid-DNA of 14 clones was prepared and sequenced. The sequence data show a distinct pattern of conserved or variable amino acid positions indicating a directed selection process (Fig. 3).

### Example 2: Expression of selected CD4 variants as a fusion protein with human IgG1

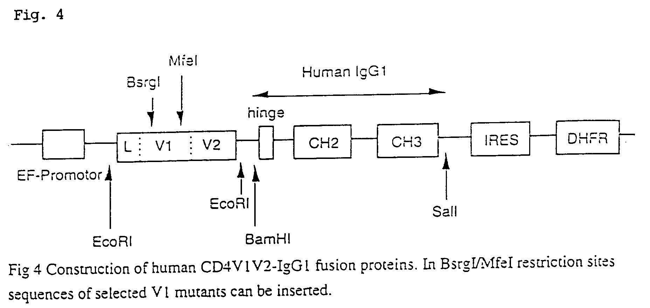

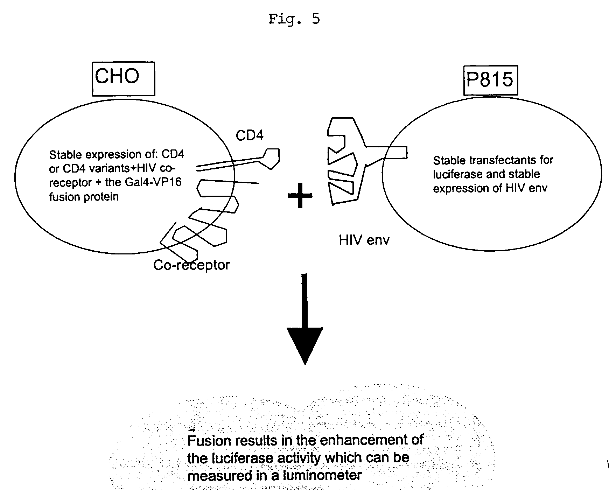

The randomized regions of the selected CD4 variants were cut with restriction enzymes BsrgI and MfeI and inserted into the V1V2 fragment of CD4 (Fig. 4). The V1V2 mutants and the wild typ V1V2 were fused to the hinge-CH2-CH3 domain of the human IgG1 according to the molecular design of the CD4-IgG1-derivative described by Byrn, Nature 344 (1989) 667-670) and cloned into the EcoRI/SaII sites of pEF-DHFR (Mack, Proc. Natl. Acad. Sci. U.S.A. 92 (1995), 7021-7025). 10⁷ CHO cells were stably transfected with 100 µg linearized plasmid DNA of the CD4 wild type or CD4 variants by means of electroporation and cultured in medium lacking nucleosides. By methotrexate-induced gene amplification according to Kaufman-RJ (Meth. Enzym. 185 (1990) 537-566) high expression levels of soluble CD4-derivatives were obtained at methotrexate concentration of 500nM.

### Example 3: Construction of an in vitro test system for inhibition of HIV env induced fusion

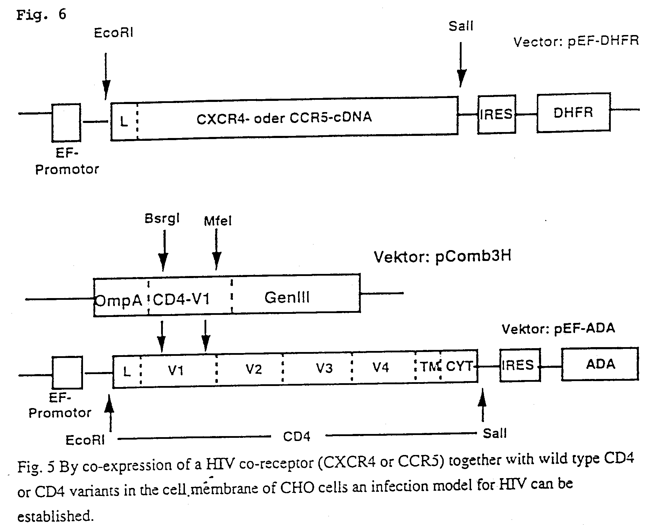

CHO cells were transfected with expression plasmids of two HIV co-receptors: CXCR4 and CCR5 (DHFR selection marker, Fig. 6). These cells were co-transfected with expression plasmids of the wild type human CD4 or CD4 molecules with replacements of the selected V1 variants. The ability of diverse HIV subtypes to infect these cells can be investigated.

In order to test the properity of wild type CD4 and its derivatives whether or to which extent they induce the membrane fusion after binding to gp120 a sensitive virus-free cell culture assay was established. In this system cells expressing the HIV receptor CD4 and the coreceptors CXCR4 or CCR5 are fused with HIV-envelope expressing cells. The fusion events are detected by the specific activation of the reporter gene luciferase which is under the expression control of the MRG5-promotor which is inducible by the transactivating fusion protein GAL4-VP16 (Sadowski-I et al., Nature. 1988 335 563-4). This means that one cellular fusion partner expresses the transactivator and the other partner carries the reporter gene.

One of the cellular fusion partners was the DHFR deficient CHO cell line (purchased from ATCC). The wild type CD4 and its derivatives were stably expressed as whole transmembrane proteins (Fig. 6) on CHO cells. For this purpose the CD4 and CD4 derivatives were cloned into the expression vector pEF-ADA derived from the expression vector pEF-DHFR (Mack, Proc. Nat. Acad. Sci. U.S.A. 92 (1995) 7021-7025)by replacing the cDNA encoding murine dihydrofolate reductase (DHFR) by that encoding murine adenosine deaminase. This plasmid was transfected for stable expression into DHFR-deficient CHO-cells by means of electroporation (Sambrook (Molecular Cloning; A Laboratory Manual, 2^{nd} edition, Cold Spring Harbour Laboratory Press, Cold Spring Harbor, NY (1989)); subsequent selection was carried out in 11-AAU-medium containing 0,1µM deoxycoformycin (dCF)as described by Kaufman-RJ (Meth. Enzym.185 (1990) 537-566) and supplemented with nucleosides. These primarily transfected CHO cells were further stably transfected with a pEF-DHFR expression plasmid (Mack, Proc. Natl. Acad. Sci. U.S.A. 92 (1995) 7021-7025) containing either CXCR4 or CCR5 which are co-receptors for T cell tropic and monocytotropic HIV isolates, respectively (for vector construction see fig. 6); selection was carried out in nucleoside-free medium All of the transfectants were additionally transfected with the fusion protein consisting of the strong transactivator VP16 and a DNA binding domain GAL4. The GAL4-VP16 encoding DNA was cloned into the eucaryotic expression vector pEF-Neo derived from the expression vector pEF-DHFR (Mack, Proc. Natl. Acad. Sci. U.S.A. 92 (1995) 7021-7025)by replacing the cDNA encoding murine dihydrofolate reductase (DHFR) by that encoding the neomycine resistance gene (Beck, Gene 19 (1982) 327-336, nucleotide position 1551 to 2345. The resulting pEF-NEO expression plasmid containing GAL4-VP16 was stably transfected by means of electroporation (Sambrook (Molecular Cloning; A Laboratory Manual, 2^{nd} edition, Cold Spring Harbour Laboratory Press, Cold Spring Harbor, NY (1989)); subsequent selection for neomycine resistance was carried out by adding G418 to the culture medium at a concentration of 1 mg/ml. Thus, all components necessary for the fusion assay were expressed stably in CHO cells.

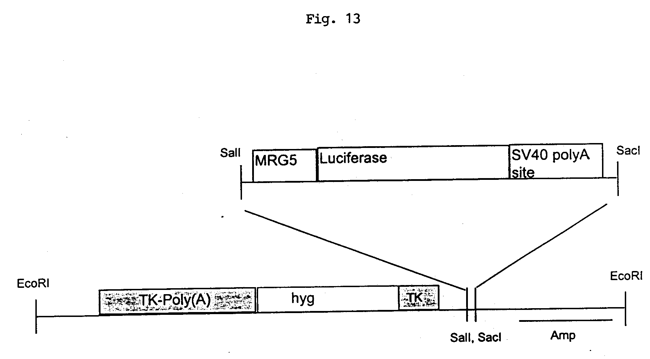

The other fusion partner was the mouse mastocytoma cell line P815. These cells express the luciferase gene under the control of the MRG5-promotor which is inducible by the fusion protein GAL4-VP16. The promotor region (=MRG5) of the luciferase gene consists of five GAL4 binding sites and a TATA box of HIV-1. A DNA fragment consisting of the MRG5-promotor, luciferase and the polyadenylation site of SV40 was cloned into the SaII and SacI sites of pHygω (Fig. 13). This vector additionally contains the hygromycine B resistance gene. This plasmid was introduced into P815 cells which were subsequently selected at 300µg/ml hygromycine B. Several cellular clones were established and tested for luciferase activity by transient transfection with the GaI4-VP16-expression plasmid described below followed by measuring the luciferase activity with a commercial kit (Berthold). Transient transfection was carried out according to standard procedures (Sambrook (Molecular Cloning; A Laboratory Manual, 2^{nd} edition, Cold Spring Harbour Laboratory Press, Cold Spring Harbor, NY (1989)). A cell clone exhibiting a high level of inducible reporter gene activation was chosen for the further work (P815 cell clone 1.2).

Sequence of the used MRG5 promotor is:

5´-
CTCGAGTAAAACGACGGCCAGTGAATTCGAGCTCGGTACCCGGGGACAGAGTCTCCGCTCGGAG
GACAGTACTCCGCTCGGAGGACAGTACTCCGCTCGGAGGACAGTACTCCGCTCGGAGGACAGTA
CTCCGCTCGGAGGACAGTACTCCGACCTGCAGGCATGCAGCTGATCTCAGATGCTGCATATAAGC
AGCTGCTTTTTGCCCGTCCTCACTCTCTTCATCCCATACCCTTCCACCAAGCTTCCC-3´.

In the cell clone 1.2 the env gene of HIV isolates LAI or MN was expressed in the neomycine resistance plasmid pEF-Neo-CTE, respectively. This vector was constructed to express the env gene in eukaryotic cells in a rev independent manner. Normally the transport of the env transcript from the nucleus into the cytoplasma is impaired and needs rev activity since HIV env contains intron sequence elements (Emerman-M et al., Cell. 1989 57(7): 1155-65). The nuclear transport and subsequent translation can be enhanced by the constitutive transport element (CTE) of Mason-Pfizer-Monkey virus (Ernst-RK et al., Mo-Cell-Biol. 1997 17(1) 135-44). The core 153 bp CTE sequence was synthesized as double-stranded oligonucleotide and cloned into the eucaryotic expression vector pEF-NEO between the neomycine resistance gene and its polyadenylation site. The transfection into P815-cells of env cloned into pEF-Neo-CTE gave rise to stable expression of env which was proved by antibody staining (flowcytometry) and the env specific fusion assay.

The inserted CTE sequence was:

5´-
GACTGGACAGCCAATGACGGGTAAGAAGATGACATTTCTCACTAACCTAAGA
CAGGAGGGCCGTCAAAGCTACTGCCTAATCCAATGACGGGTAATAGTGACA
AGAAATGTATCACTCCAACCTAAGACAGGCGCAGCCTCCGAGGGATGTGT-3´

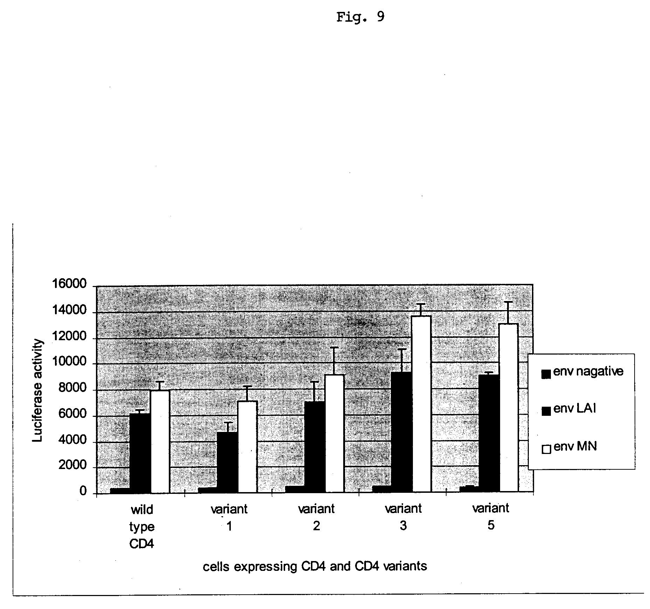

As shown in Fig.9, CD4-variant 1 (Example 1, depicted as Sequ. 1 in Fig. 3) shows reduced fusion activity with both, gp120 (env) LAI and gp120 (env) MN, compared to wild type CD4, thus indicating decreased induction of HIV-coreceptor binding sites during interaction with gp120.

### Example 4: HIV neutralization by IgG1-CD4 and its derivatives

The IgG1-CD4 and its derivatives were tested in an in vitro HIV inhibition test. The CD4 positive human T cell line H9 was used as target cells for infection with HIV isolate LAI. 10⁵ H9 cells each well were incubated in 48 well plates over night. The IgG1-CD4 and its derivatives were separately incubated in several concentrations with 100 TCID/50 of HIV-LAI for 30 minutes in 37°C. This virus solution was added to the target cells and incubated over night. 24 hours after the infection attempt the supernatant of the H9 cells was removed and refilled 3 times in order to reduce the background of HIV antigen value. 10 days after the infection attempt the HIV p24 concentration was measured in the cell supernatant by a commercial kit.

### Example 5: Purification and affinity determination of selected CD4 variants as a fusion proteins with human IgG1

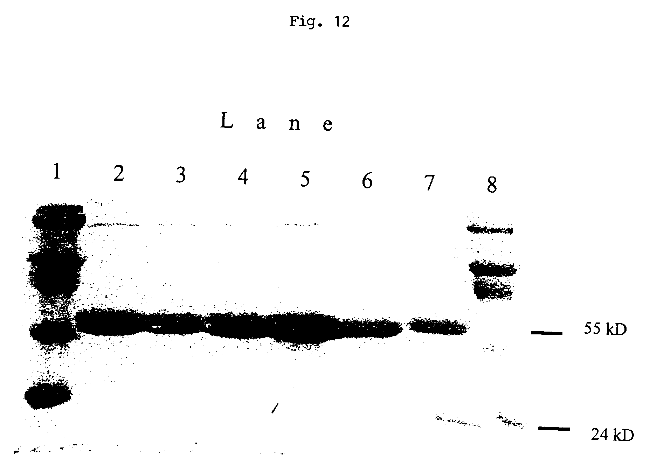

**Purification:**
CHO cells secreting the IgG1 fusion proteins of CD4- and CD4 variants (example 2) were incubated in roller bottles in a medium containing 10% dialysed FCS and 500nM methotrexate until confluence was achieved. This medium was then discarded and replaced by a medium containing 0,5% dialysed FCS and 500mM methotrexate. 1,5 liters of medium were harvested in a 9 day periode. Purification of CD4-IgG1 fusion proteins was carried out by protein A sepharose chromatography according to standard procedures. Multiple eluates of protein A-sepharose were screend in ELISA and SDS-PAGE for fractions with recombinant protein of high purity. The main pure fraction was concentrated (to approximately 1mg/ml), filtered to obtain a sterile solution and analyzed by SDS-PAGE (Figure 12).

**Surface plasmon resonance analysis:**
Affinities and kinetic constants of the interaction of CD4 and CD4-variants with gp120LAI and gp120MN were determined by surface plasmon resonance analysis using the BIACOREÆ2000 device from Pharmacia. Analysis was performed as described by (Wei-Ping Yang, J. Mol. Biol. 254.(1995)392-403). Briefly, the env proteins were immobilized in the flow chambers in different amounts. The specific binding of IgG1-CD4 and its derivatives could be demonstrated. The affinity constants (KD=off-rate/on-rate) were determined by application of several concentrations of CD4 and CD4 derivatives to the flow chambers.
As shown in Tab.3 CD4-variants 1,2,3 and 5 (depicted in Fig.3 as Sequ. 1,2,3 and 5, respectively) exhibit increased affinity to gp120LAI compared to wild type CD4 (WT CD4), with variant 3 also showing slightly increased affinity to gp120MN. Furthermore, all variants have improved on-rates to gp120LAI and gp120MN except for the interaction of variant 5 with gp120LAI.

### Example 6: Off-rate selection of CD4 variant binding to gp120LAI and gp120MN by the mean of phage display and competitive displacement by wild type IgG1-CD4

The first procedure of phage display and phage selection (Example 1) resulted in the selection of CD4-variants with a higher on-rate to gp120LAI and gp120MN compared with the wild type CD4. A new phage library was created in order to perform an off-rate selection of phages expressing CD4 variants.

For this purpose the gp120 binding domain of the CD4 variant 1 was degenerated by random primers and a phage library was created. The amino acid positions 39, 40, 46, 47 and 50 were degenerated by the NNS sequences in order to obtain all possible combinations of amino acids. The position 45 was degenerated by the sequences TWG and TGG in order to obtain the aromatic amino acids (Phenylalanine, tyrosine and tryptophan). The position 52 was degenerated by the nucleotide sequences KCA in order to obtain alanine or serine. The amino acid position 53 was degenerated by the nucleotide sequences AVC and SAS in order to obtain the amino acids: serine, threonine, asparagine, glutamic acid, aspartic acid, glutamine and histidine (see table for the used primers). The theoretical nucleic acid sequence diversity of the whole library was calculated to 1,5 x 10⁹.

**The procedure of library construction:** The 5' region of CD4 variant 1 (the dominant variant in the first phage selection procedure, depicted as Sequ. 1 in Fig.3)was PCR-amplified with the primers 5¥V1 and 3'CDR2-2. The amplified DNA species were cloned into the XhoI and SacI sites of the pComb3H. The 3' region was amplified with the primers 3'V1 together with only one of the primers 5'CDR2-2/1, 5'CDR2-2/2, 5'CDR2-2/3 or 5'CDR2-2/4 (for primer sequences see table 4). The resulting 3'fragments were subsequently cloned into the pBluescriptKS by the SpeI and ApaI sites in order to create separate 3'sublibaries in the bacteria XL1Blue. The plasmids of these separate sublibraries were isolated and precisely balanced according to the mathematical possible nucleotide sequences in order to not over-represent certain sequence motives in the whole library. The balanced 3'library was then digested with SpeI and ApaI and cloned into the already created pComB3H-5'library. 4x10⁹ transfectants were created using the electocompetent XL1-Blue bacteria purchased from Stratagene.

**Phage selection procedure:** The phage production and selection was performed as described in example 1 except for the incubation conditions described in table 5. gp120 was coated on Costar high binding A/2 plates overnight at 4∞c. The phage supernatant of 200 or 100 ml bacterial culture was harvested precipitated and resuspended in 10 ml TBS/1%BSA. 600 to 300 µl of this phage suspension was given to the wells coated with gp120LAI or gp120MN. The phage suspension was incubated 2 hours or 15 minutes in the gp120 coated wells (see table). The unbound phages were washed with TBS/0,05%tween20 solution. In the first 4 selection reactions the bound phages were eluted and used for the infection of bacteria. In the last 4 phage selection reactions after washing of the unbound phages the competitor IgG1-CD4 (that is the CD4 wild type version of the molecules described in example 2) was added into the wells. After the competition reaction the unbound phages were removed by washing with TBS/0,05%tween 20. The elution of phages was performed in two steps: 10 minutes incubation with 0,1 M HCL (pH adjusted with solid glycin to 2,2) and with one minute incubation in 5M KSCN solution. Unless otherwise mentioned, in vitro selection was carried out according to Burton, Proc. Natl. Acad. Sci. U.S.A. 88 (1991) 10134-10137.

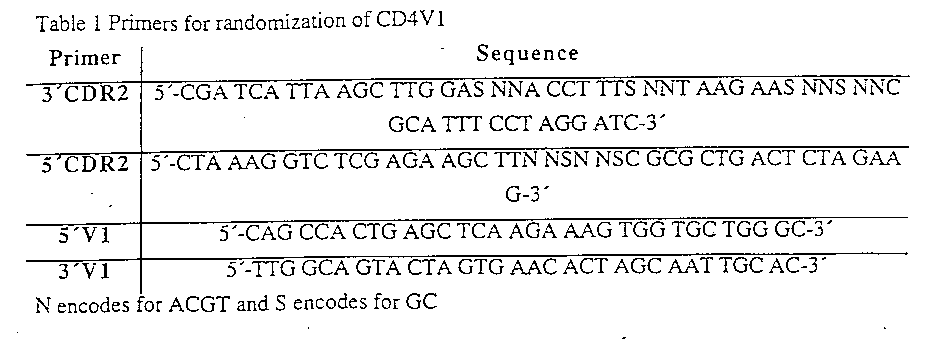

**Table 2**

| Input and output of the phage selection procedure | | | | | |
|---|---|---|---|---|---|
| frequency of Phage selection | gp120 subtype | number of pfu before selection | pfu after HCL-Elution | pfu after Rhodanid-El. | Washings n |
| 1 | LAI | 7,2x10¹¹ | 7,8x10⁷ | 8,0 x10⁵ | 1 |
| 2 | MN | 4,2 x10¹¹ | 2,0 x10⁷ | 3,3 x10⁵ | 1 |
| 3 | LAI | 1,74 x10¹¹ | 7,5 x10⁴ | 1,5 x10⁵ | 5 |
| 4 | MN | 0,18 x10¹¹ | 4,5 x10⁵ | 2,7 x10⁵ | 5 |
| 5 | LAI | 2,6 x10¹¹ | 3,5 x10⁷ | 1,0 x10⁵ | 10 |
| 6 | MN | 1,5 x10¹¹ | 6,5 x10⁶ | 2,4 x10⁴ | 10 |
| 7 | LAI | 0,9 x10¹¹ | 8,0 x10⁷ | 2,8 x10⁵ | 10 |
| 8 | MN | 2,4 x10¹¹ | 6,5 x10⁶ | 1,5 x10⁴ | 10 |
| pfu: plaque forming unit | | | | | |

**Tab 3**

| **On-rates (k**_{**on**}**), off-rates (k**_{**off**}**) and affinity constants (K**_{**D**}**) of IgG1-CD4 and its variants to gp120LAI and gp120MN** | | | | | | |
|---|---|---|---|---|---|---|
| | gp120 MN immobilisiert | | | gp120 LAI immobilisiert | | |
| **Protein** | kₒₙ M⁻¹ s⁻¹ | k_{off} s⁻¹ | K_{D} M | kₒₙ M⁻¹ s⁻¹ | k_{off} s⁻¹ | K_{D} M |
| WT CD4 | 7,64 x 10⁴ | 2,68 x 10⁻⁴ | 3,51 x 10⁻⁹ | 8,14 x 10⁴ | 9,53 x 10⁻⁴ | 11,71 x 10⁻⁹ |
| Variant 1 | 1,53 x 10⁵ | 7,52 x 10⁻⁴ | 4,92 x 10⁻⁹ | 1,49 x 10⁵ | 9,95 x 10⁻⁴ | 6,68 x 10⁻⁹ |
| Variant 2 | 1,13 x 10⁵ | 5,56 x 10⁻⁴ | 4,92 x 10⁻⁹ | 1,30 x 10⁵ | 7,99 x 10⁻⁴ | 6,15 x 10⁻⁹ |
| Variant 3 | 1,65 x 10⁵ | 5,77 x 10⁻⁴ | 3,50 x 10⁻⁹ | 1,11 x 10⁵ | 7,97 x 10⁻⁴ | 7,18 x 10⁻⁹ |
| Variant 5 | 1,05 x 10⁵ | 6,03 x 10⁻⁴ | 5,74 x 10⁻⁹ | 9,53 x 10⁴ | 8,43 x 10⁻⁴ | 8,85 x 10⁻⁹ |

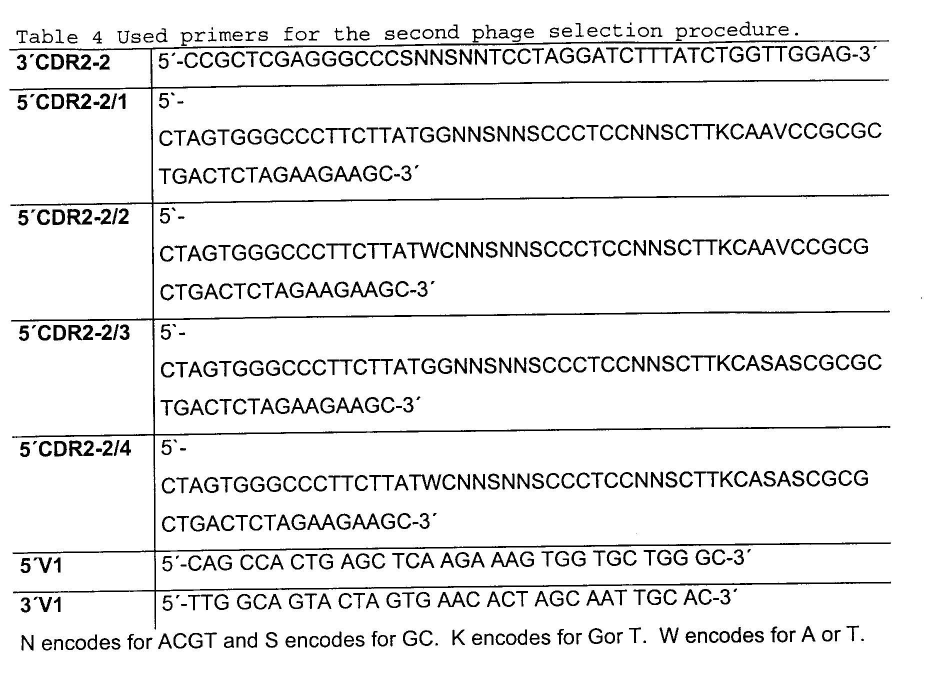

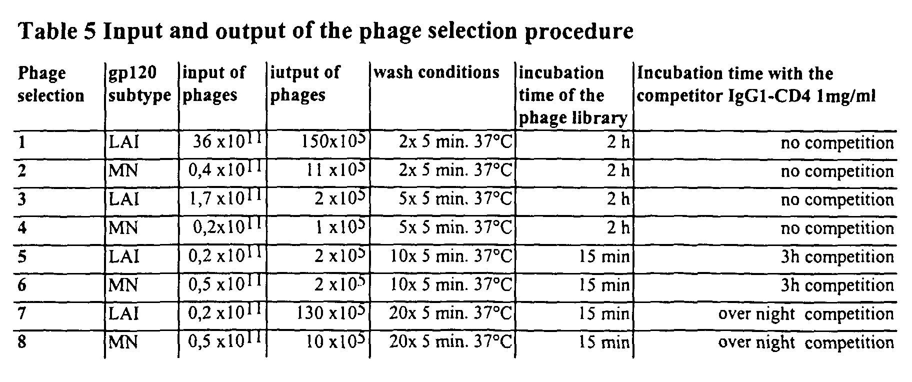

## Claims

1. A nucleic acid molecule encoding a polypeptide being derived from the human CD4 molecule but differing therefrom by
(a) a tryptophane or tyrosine residue or another aromatic amino acid instead of a threonine residue in position 45.

2. The nucleic acid molecule according to claim 1 wherein said polypeptide further differs from said human CD4 molecule by
(b) a proline residue instead of a serine residue in position 42.

3. The nucleic acid molecule according to claim 1 or 2, wherein said polypeptide further differs from said human CD4 molecule by
(c) a replacement of the amino acid asparagine in position 52 by a different amino acid.

4. The nucleic acid molecule according to claim 3 wherein said different amino acid is alanine or serine.

5. The nucleic acid molecule according to any one of claims 1 to 4 wherein said polypeptide further differs from said human CD4 molecule by
(d) a replacement of the amino acid aspartic acid in position 53 by a different amino acid, preferably glycine, serine, threonine, glutamic acid, glutamine or asparagine.

6. The nucleic acid molecule according to any of claims 1 to 5 wherein said polypeptide is further characterized by asparagine, serine or threonine in position 39, an alanine, serine or tyrosine residue in position 40, a glycine residue in position 41, arginine in position 46, a glycine residue in position 47, a proline residue in position 48, and/or glutamic acid or alanine in position 50.

7. The nucleic acid molecule according to any one of claims 1 to 6 wherein said polypeptide further differs from human CD4 in that a sequence comparable to an immunoglobulin domain hypervariable loop is inserted into at least one V-kappa-CDR homologous region of the CD4 molecule.

8. The nucleic acid molecule according to any one of claims 1 to 7 which is DNA or RNA.

9. A vector comprising the nucleic acid molecule according to any one of claims 1 to 8.

10. A host transformed with a vector according to claim 9.

11. A method of producing a polypeptide encoded by the nucleic acid according to any one of claims 1 to 8 comprising culturing the host according to claim 10 and recovering the produced polypeptide.

12. A polypeptide encoded by the nucleic acid according to any one of claims 1 to 7 or produced by the method according to claim 11.

13. A pharmaceutical composition comprising the nucleic acid according to any one of claims 1 to 8, the vector according to claim 9 and/or the polypeptide according to claim 12 and optionally a pharmaceutically acceptable carrier and/or diluent.

14. The pharmaceutical composition according to claim 13 which is a vaccine.

15. A diagnostic composition comprising the nucleic acid according to any one of claims 1 to 8, the vector according to claim 9 and/or the polypeptide according to claim 12.

16. A method of selecting a polypeptide being derived from human CD4 and having an enhanced affinity to gp120 comprising:
(a) mutating at least one amino acid not essential for the overall conformation of CD4 in the region of the polypeptide encoded by the nucleic acid molecule according to any one of claims 1 to 8 corresponding to amino acids 36 to 57 of the human CD4 molecule with the proviso that the amino acids in positions 41, 45 and 48, after the mutation step, are glycine, an aromatic amino acid and prone, respectively; and
(b) evaluating whether the affinity of the mutated polypeptide displays an increased affinity for gp120 of HIV as compared to a reference polypeptide which is preferably the polypeptide according to any one of claims 1 to 8.

17. A method of selecting a polypeptide being derived from human CD4 and having the capacity to significantly decrease and preferably avoid the induction of binding sites for HIV-co-receptors on gp120 and/or on the protein complex consisting of gp120 and said CD4-derived polypeptide comprising:
(a) mutating at least one amino acid not essential for the overall conformation of CD4 in the region of the polypeptide encoded by the nucleic acid molecule according to any one of claims 1 to 8 corresponding to amino acids 36 to 57 of the human CD4 molecule with the proviso that the amino acids in positions 41, 45 and 48, after the mutation step, are glycine, an aromatic amino acid and proline, respectively; and
(b) evaluating whether the mutated polypeptide displays the property of decreasing and preferably avoiding the induction of binding sites for HIV-co-receptors on gp120 and/or on the protein complex consisting of gp120 and said CD4-derived polypeptide as compared to a reference polypeptide which is preferably the polypeptide according to any one of claims 1 to 8.

18. The method according to claim 16 or 17 further comprising the steps of
(c) testing the effect of a sequence comparable to an immunoglobulin domain hypervariable loop which is inserted into said at least one V-kappa-CDR homologous region of the CD4 molecule of said polypeptide on the affinity and/or the decrease and preferably avoidance of the induction of binding sites for HIV-co-receptors on gp120 and/or on the protein complex consisting of gp120 and said CD4-derived polypeptide; and
(d) selecting for polypeptides having an increased affinity and/or displaying a decrease and preferably avoidance of the induction of binding sites for HIV-co-receptors on gp120 and/or on the protein complex consisting of gp120 and said CD4-derived polypeptide.

19. The method according to any one of claims 16 to 18 wherein said evaluation and/or selection steps are effected with at least two gp120 molecules obtained from different HIV isolates.

20. The method according to claim 19 wherein at least one of said gp120 molecules is gp120IIIB or gp120MN.

21. A polypeptide obtainable by the method according to any one of claims 16 to 20.
